# EUROPÄISCHE PATENTANMELDUNG

(11) **EP 4 703 005 A1**
(43) Veröffentlichungstag der Anmeldung: **04.03.2026**
(21) Anmeldenummer: 24198274.3
(22) Anmeldetag: 03.09.2024
(51) Int. Cl.: A61N 1/372, A61B 50/00

(54) **IMPLANTATVERPACKUNG, VERPACKUNGSSYSTEM EINES MEDIZINISCHEN IMPLANTATS UND VERFAHREN ZUR VERPACKUNG EINES MEDIZINISCHEN IMPLANTATS UMFASSEND EIN KONTAKTELEMENT ZUR FUNKTIONSPRÜFUNG EINER ELEKTRODE IM VERPACKTEN ZUSTAND**

(71) Anmelder: NeuroGyn AG, 6332 Baar (CH)
(72) Erfinder: Possover, Prof. Dr. Marc, 6332 Hagendorn (CH)
(74) Vertreter: Patent- und Rechtsanwälte Behrmann Wagner PartG mbB

(57) **Zusammenfassung**

Verpackungskörper für ein medizinisches Implantat, insbesondere einen Schrittmacher und/oder einen Neurostimulator, mit einer Kontaktelektrode, umfassend Verpackungsmaterial, welches entlang einer Siegelkontur des Verpackungsmaterials verlaufende Verschlussmittel umfasst, wobei wenigstens ein Teil des Verpackungsmaterials und die Verschlussmittel einen sterilen und/oder sterilisierbaren Innenraum des Verpackungskörpers begrenzen, in dem das Implantat in einem steril verpackten Zustand aufgenommen ist, wobei der Verpackungskörper, insbesondere im Innenraum, ein elektrisch leitendes Kontaktmittel aufweist, welches so angeordnet ist, dass die Kontaktelektrode des Implantats in dem steril verpackten Zustand daran zur Anlage kommt oder zur Anlage bringbar ist.

## Beschreibung

Aus dem Stand der Technik sind eine Vielzahl von medizinischen Implantaten, insbesondere Schrittmacher und/oder Neurostimulatoren, bekannt. Diese werden regelmäßig in den Körper eines Patienten implantiert und geben über eine oder mehrere bestrombare Elektroden elektrische Ströme oder Impulse an das Gewebe des Patienten ab. Die Elektroden können beispielsweise direkt an oder auf einem Gehäuse des Implantats, Schrittmachers oder Neurostimulators angeordnet oder ausgebildet sein. Alternativ kann - bevorzugt um den Implantationsort des Implantats einerseits und Abgabe- oder Stimulationsort der Elektrode andererseits freier wählen zu können - das Implantat ein Gehäuse umfassen, das eine Schnittstelle oder Anschlussmittel zur elektrischen und/oder mechanischen Kontaktierung eines Elektrodendrahts umfasst, wobei dann am gegenüberliegenden oder distalen Ende des Elektrodendrahts die Kontaktelektrode(n) angeordnet oder ausgebildet ist/sind, mit denen die elektrischen Impulse und/oder Ströme ausgegeben oder abgegeben werden. Der Elektrodendraht dient dabei in aller Regel zur Übertragung der elektrischen Signale und ist dementsprechend abseits der Kontaktelektrode nach außen hin elektrisch isoliert.

Bei bekannten Implantaten, insbesondere Schrittmachern, Neurostimulatoren oder dergleichen, ist eine verbreitete Implantationsroutine so organisiert, dass erst im Operationssaal, ggf. sogar erst im implantierten Zustand im Körper des Patienten der Elektrodendraht mit dem sonstigen Implantat, insbesondere Schrittmacher oder Neurostimulator, und/oder Gehäuse verbunden wird. Dies bedeutet auch, dass erst im Rahmen der Implantation oder ggf. sogar erst recht spät während des Implantationsvorgangs oder kurz vor Abschluss der Operation zur Implantierung eine Funktionsprüfung, insbesondere der Kontaktelektrode, insbesondere eines Elektrodendrahts, durchgeführt werden kann. Dazu werden heute bei Implantationen oder dazugehörigen Operationen Fachleute, beispielsweise des Implantatherstellers, im Operationssaal benötigt, die zusätzlich zum implantierenden Chirurgen und dessen OP-Team notwendig sind, um zum Abschluss der Implantation auch sicherzustellen, dass die Kontaktelektroden des Implantats vollständig funktionsfähig sind und in geplanter und gewollter Weise von dem Implantat angesteuert werden können.

Dies führt zu sehr aufwändigen und kostenintensiven Implantationsverfahren, weil das Operationsteam eine weitere, hochqualifizierte Fachkraft umfassen muss. Außerdem führt dies zu dem Nachteil, dass Fehlfunktionen erst sehr spät erkannt werden können, beispielsweise erst in einem Zustand, in dem der Patient einer aufwändigen und teilweise riskanten Operation unterzogen wurde. Neben dem Aufwand und dem Risiko in Zusammenhang mit der Operation führt das bekannte Vorgehen auch zu eventuell sehr kostspieligem "Ausschuss". Denn wenn erst während oder zum Abschluss der Operation/Implantation festgestellt wird, dass Implantat und/oder Kontaktelektroden nicht vollständig funktionsfähig sind, sind diese Gegenstände zumindest aus ihrer sterilen Verpackung entnommen und ggf. sogar bereits in Kontakt mit menschlichem Gewebe gekommen. Solche Geräte oder Vorrichtungen lassen sich regelmäßig nicht wieder aufarbeiten oder weiterverwenden, was nicht nur zu einer Ressourcenverschwendung führt, sondern auch die Kosten für eine dementsprechende Implantation weiter in die Höhe treiben.

Vor diesem Hintergrund besteht die Aufgabe der vorliegenden Erfindung darin, einen Verpackungskörper, ein Verpackungssystem und ein Verfahren zum Verpacken eines medizinischen Implantats vorzuschlagen, welches die Nachteile im Stand der Technik überwinden und insbesondere eine Funktionsüberprüfung der Kontaktelektroden des Implantats in einem steril verpackten Zustand des Implantats in einem Verpackungskörper ermöglicht.

Diese Aufgabe wird im Hinblick auf den erfindungsgemäßen Verpackungskörper mit den Merkmalen des Anspruchs 1 gelöst. Bezüglich des Verpackungssystems wird diese Aufgabe mit einem System gemäß Anspruch 11 gelöst. Die Aufgabe der Erfindung wird bezüglich des Verfahrens zur Verpackung eines Implantats mit den Merkmalen des Anspruchs 15 gelöst.

Vorteilhafte Ausführungsformen der vorliegenden Erfindung sind Gegenstand der nachfolgenden Beschreibung, der Figurenbeschreibung, der Figuren sowie der Unteransprüche.

Auch zur Vermeidung unnötiger Wiederholungen sollen nachfolgend alle vorrichtungsmäßig beschriebenen und offenbarten Merkmale auch als entsprechend verfahrensmäßig offenbart und beanspruchbar gelten, sowie umgekehrt.

Der erfindungsgemäße Verpackungskörper für ein medizinisches Implantat, insbesondere einen Schrittmacher und/oder einen Neurostimulator, mit einer Kontaktelektrode, welche insbesondere zur Übertragung elektrischer Signale, bevorzugt an lebendes Gewebe, eingerichtet ist, umfasst Verpackungsmaterial, welches entlang einer Siegelkontur des Verpackungsmaterials verlaufende Verschlussmittel umfasst, wobei wenigstens ein Teil des Verpackungsmaterials und die Verschlussmittel einen sterilen und/oder sterilisierbaren Innenraum des Verpackungskörpers begrenzen, in dem das Implantat in einem steril verpackten Zustand aufgenommen ist.

Erfindungsgemäß ist dabei vorgesehen, dass der Verpackungskörper, insbesondere im Innenraum, elektrisch leitende Kontaktmittel aufweist, welche so angeordnet sind, dass die Kontaktelektrode des Implantats in dem steril verpackten Zustand daran zur Anlage kommt oder zur Anlage bringbar ist. Dadurch wird ermöglicht, dass das Implantat, zumindest aber die Kontaktelektrode, in einem steril verpackten Zustand einer Funktionsprüfung unterzogen werden kann/können, weil durch einen permanenten oder herstellbaren elektrisch leitenden Kontakt zwischen der Kontaktelektrode und den Kontaktmitteln des Verpackungskörpers, insbesondere des Innenraums, die Übertragung oder Abgabe von elektrischen Signalen seitens der Kontaktelektrode und damit die Funktionalität der Kontaktelektrode - und wie weiter unten noch im Detail erläutert werden wird - ggf. auch des Implantats samt Kontaktelektrode überprüfbar ist.

Damit wird in besonders vorteilhafter Weise erreicht, dass die Funktionalität der Kontaktelektrode und ggf. auch die Wechselwirkung mit dem Implantat während des steril verpackten Zustands des Implantats überprüfbar ist, sodass beispielsweise zum Abschluss des Verpackungsvorgangs und darüber hinaus zu weiteren, kritischen Zeit-Punkten vor der endgültigen Implantation in den Körper eines Patienten die Funktionalität zumindest der Kontaktelektrode überprüfbar ist, ohne dass der steril verpackte Zustand des Implantats aufgehoben werden muss, beispielsweise dadurch, dass die Siegelkontur der Verschlussmittel oder ein sonstiger Teil des Verpackungsmaterials geöffnet werden muss.

Als Verpackungsmaterial kann beispielsweise Kunststofffolie aus mehreren Einzelschichten, beispielsweise als Mehrschicht-Laminat zum Einsatz kommen. Auch andere gut sterilisierbare Verpackungsmaterialien können Verwendung finden. Die Verschlussmittel zur Ausbildung der Siegelkontur können ebenfalls unterschiedliche ausgebildet sein. Dabei kann es sich entweder um eine Schweißverbindung des Verpackungsmaterials, mit sich selbst oder mit weiteren Materialien, handeln. Auch ein Verkleben mittels eines Haftvermittlers oder Adhäsivs kann zur Ausbildung der Verschlussmittel und der Siegelkontur dienen, sofern diese für einen Sterilisierungsprozess des versiegelten oder verschlossenen Innenraums - mitsamt des Implantats - geeignet und/oder zugelassen sind.

Wie oben bereits angedeutet, kann besonders vorteilhaft vorgesehen sein, dass die Kontaktelektrode an einem distalen Ende eines Elektronendrahts angeordnet ist. Besonders vorteilhaft können am distalen Ende des Elektrodendrahts mehrere Kontaktelektroden, beispielsweise 2, 3 oder 4 Kontaktelektroden, ausgebildet sein.

Das erfindungsgemäße Kontaktmittel des Verpackungskörpers, insbesondere im Innenraum des durch die Verschlussmittel versiegelten Innenraums, kann beispielsweise so ausgebildet sein, dass ein Teil der Kontaktelektrode oder von mehreren Kontaktelektroden oder eine vollständige Fläche der Kontaktelektrode oder der mehreren Kontaktelektroden an dem Kontaktmittel anliegt oder zur Anlage bringbar ist. Die Kontaktmittel können unterschiedliche Formen aufweisen und aus unterschiedlichen Materialien bestehen. Weiter unten wird noch auf vorteilhafte Ausführungsformen eingegangen werden. Ohne Vorgriff sei jedoch erwähnt, dass die Kontaktmittel vorteilhaft folienartig, als Scheibe, als Gelvolumen, als poröser Körper oder dergleichen ausgebildet sein können.

Gemäß einer vorteilhaften Ausführungsform der Erfindung kann vorgesehen sein, dass die Kontaktmittel so ausgebildet und/oder angeordnet sind, dass die wenigstens eine Kontaktelektrode im steril verpackten Zustand direkt oder unmittelbar und dauerhaft an dem Kontaktmittel anliegt. Bei dieser Ausführungsform handelt es sich um eine besonders einfache Ausgestaltung der Erfindung, da im Rahmen der Verpackung verhältnismäßig leicht und zuverlässig sichergestellt werden kann, dass die zumindest eine Kontaktelektrode an den Kontaktmitteln anliegt und diese Anlage auch beim Verschließen oder Versiegeln des Innenraums aufrechterhalten oder bestehen bleibt. Hierbei ist dann die größte Herausforderung die Abstimmung der Wechselwirkung zwischen der Kontaktelektrode und den Kontaktmitteln während der Anlage auf die Lebensdauer oder Haltbarkeit der sterilen Verpackung.

Denn es muss sichergestellt werden, dass während der Haltbarkeit der sterilen Verpackung des Implantats im Innenraum des Verpackungskörpers keine mechanischen, chemischen und/oder elektrochemischen Wechselwirkungen zwischen der Kontaktelektrode und dem Kontaktmittel stattfinden, die die Kontaktelektrode so beeinflussen, dass diese in ihrer Funktionalität, Qualität oder Sicherheit - insbesondere im Hinblick auf den langfristigen Verbleib in einem lebenden Körper und damit bezüglich der Biokompatibilität - nachteilig beeinflusst wird. Durch geeignete Materialwahl auf Seiten der Kontaktelektrode und der Kontaktmittel kann diese Anforderung jedoch sichergestellt oder erfüllt werden. Beispielsweise können entsprechend inerte oder unempfindliche Materialien oder zumindest Oberflächen auf Seiten der Kontaktmittel und der Kontaktelektrode verwendet werden, die auch ohne entsprechende Veränderung oder Beeinflussung über verhältnismäßig lange Zeit direkt und unmittelbar aneinander anliegen können.

In einer alternativen Ausgestaltung kann auch vorteilhaft vorgesehen sein, dass die Kontaktmittel beweglich im Innenraum angeordnet sind und in eine Kontaktposition bringbar oder überführbar sind, in welcher die Kontaktelektrode an den Kontaktmitteln anliegt, ohne dass der steril verpackte Zustand aufgehoben werden muss, insbesondere ohne dass die Siegelkontur der Verschlussmittel geöffnet werden muss.

Besonders vorteilhaft kann in diesem Zusammenhang vorgesehen sein, dass die Überführung der Kontaktmittel in die Kontaktposition von außen, bevorzugt berührungslos, durch das Verpackungsmaterial hindurch stattfindet. Beispielsweise könnte diese Überführung magnetisch stattfinden. Dazu könnte in einer vorteilhaften Ausgestaltung vorgesehen sein, dass die Kontaktmittel metallisch und/oder magnetisch ausgebildet ist, und ein von extern in die Nähe des Kontaktmittels gebrachter Permanentmagnet und/oder Elektromagnet verwendet wird, um eine Kraft auf die Kontaktmittel zu übertragen und somit die Überführung in die Kontaktposition zu bewerkstelligen.

Beispielsweise kann auf der Außenseite des Verpackungskörpers, insbesondere auf der Außenseite des Verpackungsmaterials, dazu eine Führung vorgesehen sein - zum Beispiel durch einen überstehenden Materialsteg oder mehrere überstehende Materialstege -, die die Führung des externen Magneten vorgeben und damit erleichtern oder sicherstellen, dass die Kontaktmittel im Innenraum des Verpackungskörpers in die Kontaktposition überführt werden, ohne dass der steril verpackte Zustand des Verpackungskörpers aufgehoben werden muss.

Gegebenenfalls kann zu diesem Zweck auch im Innenraum vorgesehen sein, dass Führungsmittel oder Führungskonturen nach innen in den Innenraum des Verpackungskörpers vorstehen oder überstehen, die die Führung der Kontaktmittel bereitstellen oder bewerkstelligen.

Alternativ zu einer magnetisch verursachten Überführung der Kontaktmittel in die Kontaktposition kann beispielsweise auch eine alternative Ausführung realisiert sein. Beispielsweise ist es möglich, die Verpackung oder den Verpackungskörper so zu gestalten, dass dieser verformbar, beispielsweise knickbar oder biegbar, ist, ohne dass dadurch der steril verpackte Zustand aufgehoben wird, wobei gleichzeitig die besagte Verformung, beispielsweise das Knicken, des Verpackungskörpers dazu führt, dass im Inneren oder im Innenraum des Verpackungskörpers die Kontaktmittel zwangsgeführt in die Kontaktposition überführt werden.

In einer weiteren, besonders vorteilhaften Ausgestaltung kann vorgesehen sein, dass durch eine entfernbare Trenn- oder Schutzschicht, die zwischen Kontaktelektrode und Kontaktmitteln angeordnet ist, und die so ausgeführt ist, dass eine Entfernung und daraus resultierende Anlage der Kontaktmittel an der Kontaktelektrode möglich ist, ohne dass der steril verpackte Zustand aufgehoben werden muss.

Die bewegliche Ausgestaltung der Kontaktmittel, wie auch das alternative oder zusätzliche Vorsehen einer entfernbaren Trenn- oder Schutzschicht zwischen den Kontaktmitteln und der Kontaktelektrode, hat den Vorteil, dass nicht permanent oder dauerhaft ein Kontakt zwischen Kontaktelektrode und Kontaktmitteln bestehen muss, was die oben beschriebenen Herausforderungen mit sich bringt. Gleichzeitig haben diese Ausführungsform als wesentliche Herausforderung die Herstellung des Kontakts zwischen Kontaktmittel und Kontaktelektrode ohne Aufheben oder Öffnen des sterilen Zustands des Verpackungskörpers. Auch bei der vorteilhaften Trenn- oder Schutzschicht kann vorteilhaft vorgesehen sein, dass diese so angeordnet ist, dass eine von außen vorgenommene Verformung, z.B. Knicken oder Biegen, des Verpackungskörpers möglich ist, die zwar einerseits die Trenn- oder Schutzschicht zwischen den Kontaktmitteln und der Kontaktelektrode entfernt, gleichzeitig jedoch die Sterilität oder den sterilen Zustand des Verpackungskörpers aufrechterhält. Die Trenn- oder Schutzschicht kann beispielsweise als elektrisch isolierende, beispielsweise aus Kunststoff hergestellte, Fahne ausgebildet sein, die an einer Seite an dem Verpackungsmaterial befestigt ist, sodass eine bereichsweise Verformung des Verpackungsmaterials dazu führt, dass die Trenn- oder Schutzschicht im Innenraum ebenfalls eine Verlagerung oder Bewegung erfährt.

In einer weiteren, besonders vorteilhaften Variante des Verpackungskörpers kann vorgesehen sein, dass der Verpackungskörper Führungsmittel, insbesondere Führungskonturen, aufweist, die die Lage und Ausrichtung des Implantats und/oder der Kontaktelektrode im verpackten Zustand vorgeben. Bevorzugt können die Führungskonturen die Lage eines Gehäuses des Implantats und eines Elektrodendrahts mitsamt der Kontaktelektrode vorgeben.

Dadurch wird sichergestellt, dass das Implantat und insbesondere die Kontaktelektrode im steril verpackten Zustand möglichst wohldefinierte Zustände und/oder Positionen einnehmen, sodass auch die entsprechende Anordnung, Positionierung und/oder Bewegung der Kontaktmittel entsprechend gut und sicher auf die Kontaktelektrode abgestimmt und/oder zugeschnitten werden können, so dass dann zuverlässig der elektrische Kontakt durch die Anlage hergestellt wird oder herstellbar ist. Die Führungsmittel oder Führungskonturen sind bevorzugt im Innenraum des Verpackungskörpers ausgebildet. Beispielsweise können die Führungsmittel als konturiertes Einlegeteil oder als mit dem Verpackungsmaterial verbundene oder als aus dem Verpackungsmaterial, insbesondere monolithisch, geformte oder umgeformte Strukturen ausgebildet sein.

In einer weiteren, besonders vorteilhaften Variante des Verpackungskörpers kann vorgesehen sein, dass Anpressmittel vorgesehen sind, die im steril verpackten Zustand die Kontaktelektrode in Richtung der Kontaktmittel kraftbeaufschlagen. Auch die Anpressmittel können zum Beispiel als Einlegeteil, als mit dem sonstigen Verpackungsmaterial verbundenes Formteil oder als monolithisch aus dem Verpackungsmaterial ausgebildeten Bestandteil des Verpackungsmaterials ausgeführt sein. Die Anpressmittel können in besonders vorteilhafter Weise an die Verschlussmittel und/oder die Siegelkontur angepasst sein, sodass beim Ausbilden der Siegelkontur eine Anpresswirkung der Kontaktelektrode in Richtung der Kontaktmittel erzeugt wird. Die Anpresskraft kann dabei vorteilhaft darauf abgestimmt werden, ob eine dauerhafte oder nicht dauerhafte, unmittelbare und direkte Anlage zwischen den Kontaktmitteln und der Kontaktelektrode besteht. Beispielsweise kann ein in den Innenraum vorstehendes Podest des Verpackungsmaterials als Anpressmittel dienen.

Eine weitere, besonders wünschenswerte Ausgestaltung des Verpackungskörpers sieht vor, dass die Kontaktmittel metallisches Material umfassen. Wie oben bereits angesprochen, kann über metallisches Material auf Seiten der Kontaktmittel eine magnetisch induzierte Verlagerung oder Bewegung des Kontaktmittels verursacht werden. Auch ermöglicht die wenigstens teilweise metallische Ausbildung der Kontaktmittel die elektrische Kontaktierung der Kontaktelektrode und damit die Funktionsprüfung der Kontaktelektrode von außen im steril verpackten Zustand.

Die Kontaktmittel können beispielsweise als metallisches Plättchen oder als metallische Scheibe ausgebildet sein. Auch ein mit einer Metallbeschichtung versehenes Kunststoffelement kann vorteilhaft zum Einsatz kommen. Beispielsweise kann auch ein mit einem metallischen Werkstoff beschichteter Teil des Verpackungsmaterials selbst die Kontaktmittel ausbilden oder bereitstellen.

Alternativ zu einem metallischen oder Metall umfassenden Material können jedoch auch andere elektrisch leitfähige Materialien zum Einsatz kommen. Hier könnten beispielsweise Gele, Pasten, Fasermaterialien, poröse Materialien oder dergleichen verwendet werden, sofern diese eine gewisse elektrische Leitfähigkeit aufweisen. Dies können ebenfalls plättchenförmig ausgebildet sein, wobei dann - auch bei einer metallischen Ausführung - die Kontaktelektrode vorteilhaft an einer Oberfläche zur Anlage kommt. Alternativ kann auch eine Ausbildung als Volumenkörper, zum Beispiel als Kugel, Quader oder dergleichen vorgesehen sein, wobei dann die Kontaktelektrode bei der Anlage bevorzugt in das Volumen der Kontaktmittel hineinragt oder hingesteckt ist/wird.

In einer besonders vorteilhaften Weiterbildung kann vorgesehen sein, dass die Kontaktmittel eine Leitfähigkeit aufweisen, die der vom lebenden, insbesondere menschlichen, Gewebe entsprechen oder wenigstens im Wesentlichen entsprechen. Dadurch kann in besonders vorteilhafter Weise bei der Funktionsprüfung der Kontaktelektrode ein Zustand bereitgestellt werden, der im größtmöglichen Maße dem endgültig implantierten Zustand der Kontaktelektrode entspricht.

Beispielsweise über die Zusammensetzung der Oberfläche der Kontaktmittel oder die Materialzusammensetzung der Kontaktmittel kann die Leitfähigkeit entsprechend eingestellt oder vorgegeben werden.

In einer Weiterbildung des Verpackungskörpers kann zudem besonders vorteilhaft vorgesehen sein, dass die Kontaktmittel, insbesondere optische, Leitfähigkeit-Anzeigemittel umfassen, die eine Abweichung von einem vorgegebenen oder ursprünglichen Leitwert der Kontaktmittel anzeigen oder erkennbar machen. Dadurch kann sichergestellt werden, dass im Zeitpunkt der Funktionsprüfung der Kontaktelektrode erkennbar ist, ob und ggf. inwieweit der ursprüngliche oder vorgegebene Leitwert der Kontaktmittel noch gegeben ist oder eingehalten wird. Die Leitfähigkeit-Anzeigemittel können beispielsweise durch eine Färbung oder Verfärbung der Kontaktmittel bereitgestellt oder realisiert werden. Hierzu können beispielsweise Farbstoffe eingesetzt werden, die in Abhängigkeit von Feuchtigkeit oder elektrischem Widerstand eine Verfärbung oder einen Farbumschlag durchführen.

Weiterhin kann, wie oben bereits angedeutet, vorgesehen sein, dass die Kontaktmittel so ausgebildet sind, dass mehrere, insbesondere alle, Einzelelektroden der Kontaktelektrode im steril verpackten Zustand in Kontakt mit den Kontaktmitteln sind oder in Kontakt bringbar sind. Dadurch wird erreicht, dass alle Einzelelektroden der Kontaktelektrode im Hinblick auf Funktionalität überprüfbar sind.

Die oben genannte Aufgabe wird auch durch ein Verpackungssystem zur sterilen Verpackung eines medizinischen Implantats umfassend das medizinische Implantat, insbesondere einen Schrittmacher und/oder einen Neurostimulator, mitsamt einer Kontaktelektrode, insbesondere zur Übertragung elektrischer Signale, bevorzugt an lebendes Gewebe, gelöst, bei dem erfindungsgemäß vorgesehen ist, dass ein Verpackungskörper nach einer der vorangehend offenbarten Ausführungsformen vorgesehen ist. In dem Verpackungssystem liegen dann im steril verpackten Zustand das Implantat mitsamt Kontaktelektrode im Innenraum des Verpackungskörpers vor.

Durch die entsprechende Anordnung der Kontaktmittel in Bezug auf die Kontaktelektrode sind Kontaktmittel und Kontaktelektrode entweder dauerhaft und permanent im direkten oder unmittelbaren Kontakt zueinander oder können entsprechend, wie oben bereits ausführlich beschrieben, miteinander zur Anlage gebracht werden, ohne dass die sterile Verpackung oder der steril verpackte Zustand aufgebrochen oder aufgehoben werden muss. Somit kann mit dem erfindungsgemäßen Verpackungssystem die Funktionsfähigkeit der Kontaktelektrode sowohl nach dem Beenden des Verpackungsprozesses und dem Sterilisieren des Verpackungskörpers als auch vor der Operation zur Implantation des Implantats überprüft werden, ohne dass dazu oder dabei die Sterilität des Verpackungskörpers beendet oder aufgehoben werden muss.

Dies erlaubt insbesondere, dass bei einer Feststellung oder Detektion eines Fehlers oder einer nicht vollständigen, eingeschränkten oder nicht vorhandenen Funktionalität der Kontaktelektrode diese mitsamt dem Implantat in dem nach wie vor steril versiegelten Verpackungskörper ausgetauscht und ggf. an den Hersteller zurücküberführt werden kann. In diesem Fall kann beispielsweise ein Ersatzimplantat, ebenfalls in einer entsprechenden Verpackung oder einem entsprechenden Verpackungskörper für die Implantation genutzt werden, nachdem für dieses Ersatzimplantat die Funktionsfähigkeit der Kontaktelektrode - abermals vorteilhaft im nach wie vor steril verpackten Zustand - sichergestellt oder bestätigt wurde.

In einer vorteilhaften Ausführungsform des Verpackungssystems kann vorgesehen sein, dass im steril verpackten Zustand die Kontaktelektrode elektrisch leitend, und insbesondere mechanisch, besonders bevorzugt funktionsbereit, mit dem Implantat verbunden ist. Dies führt in besonders vorteilhafter Weise dazu, dass nicht nur die Funktionsfähigkeit der Kontaktelektrode überprüfbar ist, sondern dass in vorteilhafter Weise auch die gesamte Funktionalität des Implantats mitsamt der Funktion der verbundenen Kontaktelektrode überprüfbar ist. Dies erlaubt in besonders vorteilhafter Weise eine Gesamtüberprüfung des medizinischen Implantats im steril verpackten Zustand des Verpackungskörpers. Außerdem hat dies den besonderen Vorteil, dass ggf. die zusätzliche Fachperson im Rahmen der Implantation des Implantats, wie oben im Rahmen der Einleitung beschrieben, vollständig entfallen kann oder überflüssig wird.

Denn durch eine vorherige mechanische und/oder elektrische, insbesondere funktionsbereite Verbindung von Implantat und Kontaktelektrode und die dazugehörige Funktionsprüfung noch im verpackten Zustand kann der Hersteller und/oder Verpacker des Implantats seinerseits unabhängig vom Implantationsvorgang die Funktionalität von Implantat und Kontaktelektrode testen und vor Versand oder Auslieferung sicherstellen. Das Implantationsteam oder Operationsteam, insbesondere der Operateur, kann dann entweder ohne weitere Prüfung die Implantation des Implantats vornehmen oder aber vor unwiderruflichem Aufheben des steril verpackten Zustands des Verpackungskörpers abermals sicherstellen, dass Implantat und Kontaktelektrode vollständig funktionsfähig sind, wobei diese Überprüfung besonders vorteilhaft dadurch durchgeführt werden kann, dass einerseits das Implantat mit der Kontaktelektrode funktionsfähig verbunden ist und andererseits dadurch, dass im steril verpackten Zustand die Funktionalität der Kontaktelektrode durch die Anlage zu den im Innenraum des Verpackungskörpers angeordneten Kontaktmitteln überprüfbar ist.

In einer weiteren, vorteilhaften Ausgestaltung des Verpackungssystems umfasst dieses eine Kontrolleinrichtung, mit einer Drahtloskommunikationsschnittstelle, die zum Aufbau einer Kommunikationsverbindung mit dem Implantat, bevorzugt über eine Drahtloskommunikationsschnittstelle des Implantats eingerichtet ist. Dies ermöglicht in besonders vorteilhafter Weise, dass im steril verpackten Zustand des Verpackungskörpers in unterschiedlichsten Stufen oder Stadien des Herstellungs- und Verwendungsprozesses des Implantats, insbesondere nach der Herstellung des steril verpackten Zustands, die Funktionalität von Implantat und/oder Kontaktelektrode überprüfbar ist, ohne dass die Sterilität des Implantats und/oder der Kontaktelektrode beeinträchtigt wird. Dies erhöht in großem Umfang die Zuverlässigkeit, dass nur funktionsfähige Implantate und Kontaktelektroden in die Operationssäle zur Implantation gelangen und dass eine Implantation oder eine entsprechende Operation nur durchgeführt wird, wenn das Operationsteam oder der Operateur sicher weiß, dass das vorliegende Implantat mitsamt der Kontaktelektrode funktionsfähig ist.

Die Drahtlos-Kommunikationsschnittstellen auf Seiten der Kontrolleinrichtung sowie auf Seiten des Implantats können entweder durch standardisierte Übertragungsfrequenzen und Übertragungsprotokolle, wie beispielsweise NFC, Bluetooth, WLAN, LTE, 5G oder dergleichen verwirklicht werden. Alternativ ist es auch möglich, ein eigenständiges Drahtlos-Kommunikationsprotokoll und dazugehörige Hardware zu verwenden.

Gemäß einer weiteren, besonders vorteilhaften Ausführungsform des Verpackungssystems kann vorgesehen sein, dass die Kontrolleinrichtung zur Funktionsüberprüfung des Implantats einschließlich einer Funktionsüberprüfung der Kontaktelektrode bei bestehender Kommunikationsverbindung mit dem Implantat eingerichtet ist.

Die oben genannte Aufgabe wird auch durch ein Verfahren zur sterilen oder sterilisierbaren Verpackung eines Implantats gelöst, bei dem in einem Verfahrensschritt ein Anordnen des Implantats, insbesondere eines Schrittmachers und/oder eines Neurostimulators, mit einer Kontaktelektrode zur Übertragung elektrischer Signale, bevorzugt an lebendes Gewebe, in einem Verpackungsmaterial stattfindet. In einem weiteren Verfahrensschritt erfolgt das Ausbilden einer Siegelkontur im Verpackungsmaterial über Verschlussmittel, wodurch wenigstens ein Teil des Verpackungsmaterials und die Verschlussmittel einen sterilen oder sterilisierbaren Innenraum des Verpackungskörpers begrenzen, in dem das Implantat, insbesondere mitsamt Kontaktelektrode, aufgenommen ist.

Erfindungsgemäß ist dabei vorgesehen, dass die Kontaktelektrode des Implantats so bezüglich des Verpackungskörpers, insbesondere im Innenraum, angeordnet und/oder ausgerichtet ist, dass in einem steril verpackten Zustand der Verpackungskörper ein elektrisch leitendes Kontaktmittel des Verpackungskörpers an der Kontaktelektrode zur Anlage bringt oder zur Anlage bringbar ist.

Bezüglich der Vorteile und vorteilhaften Wirkungen des Verfahrens wird auf das oben beschriebene Verpackungssystem sowie den Verpackungskörper verwiesen.

Gemäß einer vorteilhaften Ausführungsform des Verfahrens kann vorgesehen sein, dass die Kontaktelektrode im steril verpackten Zustand des Verpackungskörpers mit dem Implantat elektrisch leitend und insbesondere mechanisch, besonders bevorzugt funktionsbereit, mit dem Implantat verbunden wird, bevor die Siegelkontur über die Verschlussmittel ausgebildet wird. Dies stellt sicher, dass im steril verpackten Zustand nicht nur eine Funktionalität oder Funktionsfähigkeit der Kontaktelektrode, sondern des gesamten Implantats mitsamt der Kontaktelektrode durchführbar ist, was vorteilhaft drahtlos und von außen durchgeführt werden kann.

Dementsprechend sieht eine weitere, besonders wünschenswerte Ausgestaltung des Verfahrens vor, dass im steril verpackten Zustand eine Funktionsüberprüfung des Implantats einschließlich einer Funktionsüberprüfung der Kontaktelektrode mittels einer drahtlos mit dem Implantat verbundenen Kontrolleinrichtung durchgeführt wird. Diese Funktionsüberprüfung kann bevorzugt mehrfach vor dem Öffnen des Verpackungskörpers und dem Beenden des steril verpackten Zustandes zu verschiedenen Anlässen, beispielsweise zum Abschluss der Verpackung, und wenigstens zur Vorbereitung der Implantation durchgeführt werden. Besonders bevorzugt kann dabei die Kontrolleinrichtung so ausgebildet sein, dass eine im Wesentlichen vollautomatische Funktionsüberprüfung durchgeführt oder ausgeführt wird, sodass auch ein weniger geschultes Personal, beispielsweise ein Mitglied des Operationsteams oder der Operateur selber, die Funktionsprüfung des Implantats mitsamt der Kontaktelektrode unmittelbar vor der Implantation und der Aufhebung des steril verpackten Zustands durchführen kann.

Nachfolgend wird die vorliegende Erfindung anhand von lediglich schematischen, vorteilhafte Ausführungsbeispiele zeigende Zeichnungen beschrieben.

Darin zeigen:
- Fig. 1:: einen erfindungsgemäßen Verpackungskörper in einem unverschlossenen Zustand;
- Fig. 2:: ein Verpackungssystem aus einem Verpackungskörper und einem Implantat mitsamt Kontaktelektrode in einem steril verpackten Zustand gemäß einer ersten Ausführungsform;
- Fig. 3:: ein Verpackungssystem aus einem Verpackungskörper und einem Implantat mitsamt Kontaktelektrode in einem steril verpackten Zustand gemäß einer zweiten Ausführungsform;
- Fig. 4:: ein Verpackungssystem aus einem Verpackungskörper und einem Implantat mitsamt Kontaktelektrode in einem steril verpackten Zustand gemäß einer dritten Ausführungsform;
- Fig. 5:: ein beispielhafter Verfahrensablauf zur Durchführung des erfindungsgemäßen Verfahrens.

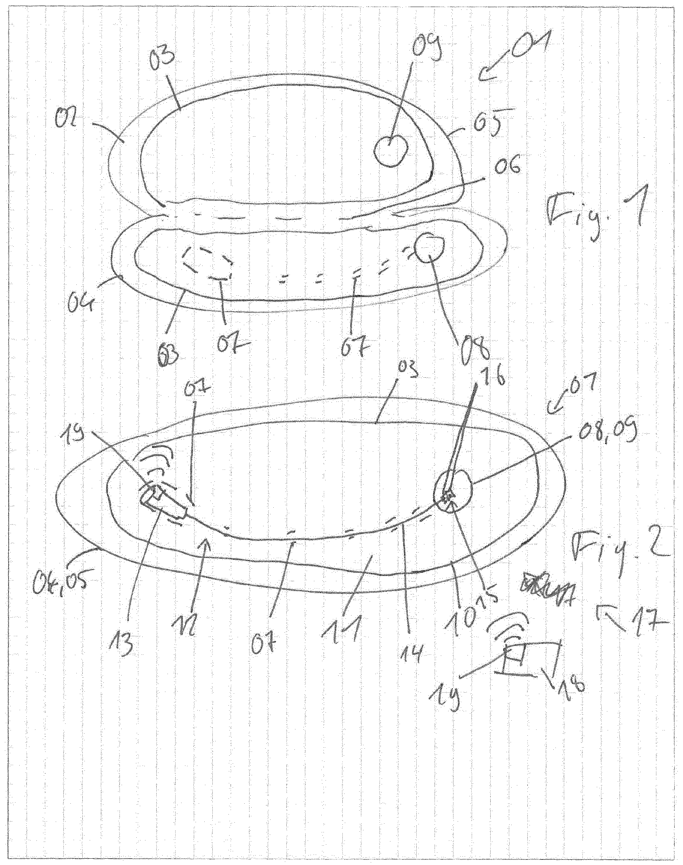

Die Fig. 1 zeigt einen Verpackungskörper 01 für ein Implantat, insbesondere einen Schrittmacher oder einen Neurostimulator. In der Fig. 1 ist lediglich der Verpackungskörper 01 dargestellt, nicht jedoch das Implantat. In diesem Zusammenhang wird auf die Darstellung des Verpackungssystems in den Fig. 2 bis 4 verwiesen.

Der Verpackungskörper 01 kann beispielsweise ein folienartiges Verpackungsmaterial 02 aufweisen, welches Verschlussmittel 03 zur Ausbildung einer Siegelkontur umfassen. Da die Fig. 1 einen offenen oder unverschlossenen Zustand des Verpackungskörpers 01 zeigt, sind die Verschlussmittel 03 noch nicht zur Siegelkontur verbunden oder verschlossen.

Im Beispiel der Fig. 1 kann der Verpackungskörper einen Bodenteil 04 und einen Deckelteil 05 umfassen, die bevorzugt in einem Übergangsbereich 06 miteinander verbunden sind. Der Übergangsbereich 06 kann auch ein monolithisch mit dem Bodenteil 04 und dem Deckelteil 05 ausgebildeter Teil des Verpackungsmaterials 02 sein. Beispielsweise kann in dem Bodenteil 04 entweder als separates Einlegeteil, als mit dem Verpackungsmaterial 02 verbundenes Material oder als monolithisch aus dem Verpackungsmaterial 02 ausgebildetes Führungsmittel 07 vorgesehen sein, die die Position und Anordnung eines Implantats und einer Kontaktelektrode im verpackten und/oder sterilen Zustand des Verpackungskörpers vorgeben. Die Führungsmittel 07 können als nach Innen vorstehende durgängige oder unterbrochene Stege realisiert sein. Weiterhin ist vorgesehen, dass beispielsweise im Bodenteil 04 Kontaktmittel 08 realisiert sind, die im steril verpackten Zustand des Verpackungskörpers 01 dafür sorgen, dass eine Kontaktelektrode des Implantats dauerhaft, permanent und unmittelbar oder direkt an den Kontaktmitteln 08 anliegen oder aber zur Anlage bringbar sind, wie mit Bezug zu den Fig. 2 bis 4 noch im Detail beschrieben.

In dem Deckelteil 05 des Verpackungskörpers 01 sind zudem Anpressmittel 09 realisiert, die so positioniert und ausgerichtet sind, dass beim Verschließen des Verpackungskörpers 01 und beim Ausbilden einer Siegelkontur entlang der Verschlussmittel 03 eine Kontaktelektrode in Richtung der Kontaktmittel 08 kraftbeaufschlagt wird. Im Beispiel der Fig. 1 kann der Verschluss und das Ausbilden der Siegelkontur entlang der Verschlussmittel 03 beispielsweise dadurch hergestellt werden, dass das Bodenteil 04 und das Deckelteil 05 zueinander verschwenkt oder geklappt werden, sodass die Verschlussmittel 03 im Wesentlichen deckungsgleich aufeinander zur Anlage kommen. Die Anpressmittel können zum Beispiel als Schaumstoffkörper ausgebildet sein. Auch ein anderer wenigstens teilweise elastisch verformbarer Körper, der eine Anpresskraft auf die Kontaktelektrode im steril verpackten Zustand ausüben kann, kann verwendet werden, soweit er sich für eine Sterilisation eignet und keine schädlichen Wechselwirkungen durch den Kontakt mit oder an der Kontaktelektrode hervorruft.

Fig. 2 zeigt den verschlossenen und versiegelten Verpackungskörper 01 der Ausführungsform der Fig. 1 in einer transparenten Draufsicht. Es ist zu erkennen, dass Bodenteil 04 und Deckelteil 05 im Wesentlichen deckungsgleich aufeinandergelegt sind und dass mit den Verschlussmitteln 03 eine Siegelkontur 10 ausgebildet wurde, die einen Innenraum 11 umschließen. Im Innenraum 11 ist ein Implantat 12 angeordnet, welches über ein Gehäuse 13 und ein Elektrodendraht 14 verfügt, wobei am distalen Ende des Elektrodendrahts 14 eine Kontaktelektrode 15 ausgebildet ist, die beispielsweise mehrere Einzelelektroden 16 umfassen kann. In der Draufsicht der Fig. 2 ist erkennbar, dass die Kontaktmittel 08 und die Anpressmittel 09 im Wesentlichen deckungsgleich zueinander angeordnet sind. Das Implantat 12 wird in seiner Position und Ausrichtung durch die Führungsmittel 07 definiert oder positioniert. Die Verschlussmittel 03 und die Siegelkontur 10 sind vorteilhaft so auf die Kontaktmittel 08, die Anpressmittel 09 und die Kontaktelektrode 15 abgestimmt, dass eine dauerhafte und direkte oder unmittelbar elektrisch leitende Kontaktierung zwischen der Kontaktelektrode 15 und den Kontaktmitteln 08 im verschlossenen oder versiegelten Zustand des Verpackungskörpers 01 erzeugt wird.

Wie in der Fig. 2 dargestellt, kann für das Verpackungssystem 17 vorteilhaft vorgesehen sein, dass die Kontaktelektrode 15 elektrisch leitend und insbesondere mechanisch, besonders bevorzugt funktionsbereit, mit dem restlichen Implantat 12, insbesondere mit dem Gehäuse 13, verbunden ist. Dadurch kann erreicht werden, dass in dem verschlossenen Zustand des Verpackungskörpers 01 und dem damit erreichten sterilen oder sterilisierbaren Verpackungszustand des Implantats 12 die Funktionsfähigkeit der Kontaktelektrode 15 und des sonstigen Implantats 12 mit einer externen Kontrolleinrichtung 18 überprüfbar ist. Dazu kann über jeweils eine Drahtloskommunikationsschnittstelle 19 auf Seiten der Kontrolleinrichtung 18 sowie auf Seiten des Implantats 12 eine Kommunikation stattfinden, die einerseits das Implantat 12 zur Durchführung eines Kontrollmodus auffordert oder anregt und nach Abschluss des Kontrollmodus entsprechende Kontrolldaten über die Funktionsfähigkeit zurück an die Kontrolleinrichtung 18 übermittelt. Die benötigte elektrische Energie kann auf Seiten des verpackten Implantats 12 entweder durch eine im Implantat vorgesehene Batterie oder einen Akkumulator bereitgestellt werden oder alternativ zusammen mit der Kommunikation drahtlos über die Drahtloskommunikationsschnittstelle 19 von der Kontrolleinrichtung 18 an das Implantat 12 übertragen werden.

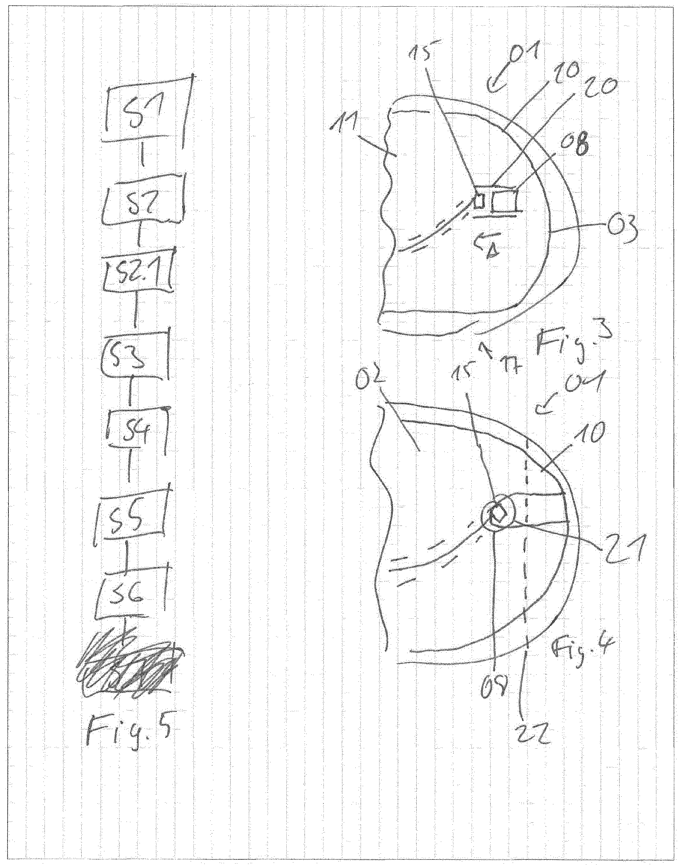

Die Fig. 3 zeigt einen Ausschnitt aus einer alternativen Ausführungsform eines erfindungsgemäßen Verpackungssystems 17 mit einem Verpackungskörper 01. In dieser Ausführungsform ist vorgesehen, dass die Kontaktmittel 08 beweglich ausgebildet sind und erst nach einer Bewegung, beispielsweise entlang des Richtungspfeils A, die Kontaktmittel 08 mit der Kontaktelektrode 15 des Implantats zur Anlage kommen. Die Verlagerung oder Bewegung der Kontaktmittel 08 kann von außen induziert werden, bevorzugt ohne die Siegelkontur 10 der Verschlussmittel 03 zu öffnen und damit ohne den steril verpackten Zustand zu beenden. Die Verlagerung oder Bewegung der Kontaktmittel 08 kann beispielsweise von außen über eine magnetische Wechselwirkung mit einem Dauermagneten oder Elektromagneten erreicht werden.

Um die Bewegung der Kontaktmittel 08 zu limitieren oder vorzugeben, können im Innenraum 11 des Verpackungskörpers 01 weitere Führungskonturen 20 vorgesehen sein. In dieser Ausführungsform ist die Anlage zwischen Kontaktelektrode 15 und Kontaktmitteln 08 vorteilhaft nur temporär und kann beispielsweise explizit zu Zeitpunkten hergestellt oder herbeigeführt werden, in denen die Funktionsüberprüfung, wie mit Bezug zu Fig. 2 skizziert, durchgeführt wird. Auch auf der Außenseite des Verpackungskörpers 01 können dazugehörige Führungskonturen ausgebildet sein, die die Bewegung des Magneten vorgeben. Diese sind in der Fig. 3 nicht dargestellt.

In der Darstellung der Fig. 4 ist eine Ausführungsform gezeigt, bei der zwischen der Kontaktelektrode 15 und den Kontaktmitteln 08 in einem steril verpackten Zustand und ohne eine von außen induzierte, beabsichtigte Verformung des Verpackungskörpers 01 eine Trenn- oder Schutzschicht angeordnet ist. Die Trenn- oder Schutzschicht 21 kann beispielhaft als dünne Fahne oder Folie ausgebildet sein und beispielsweise mit dem Verpackungsmaterial 02 oder der Siegelkontur 10 verbunden sein. Der Verpackungskörper 01 kann eine Sollverformungslinie 22 aufweisen, die beispielsweise durch optische Markierungen für den Benutzer erkennbar ist und/oder durch entsprechende Materialveränderungen oder Gefügeveränderungen im Material des Verpackungskörpers 01 ausgebildet ist und die es erlaubt, den Verpackungskörper 01 zu verformen, ohne den steril verpackten Zustand aufzuheben oder zu beeinträchtigen.

Wie in der Fig. 4 skizziert, kann jedoch eine Verformung relativ zur Sollverformungslinie 22 dafür sorgen, dass die Trenn- oder Schutzschicht 21 zwischen Kontaktelektrode 15 und den Kontaktmitteln 08 entfernt wird und somit eine Anlage zwischen Kontaktelektrode 15 und Kontaktmitteln 08 hergestellt wird, sodass dann eine elektrisch leitende Verbindung zwischen Kontaktmitteln 08 und Kontaktelektrode 15 besteht, aufgrund derer abermals eine Funktionsprüfung des Implantats 12 mitsamt Kontaktelektrode 15 durchführbar ist.

In der Fig. 5 wird beispielhaft ein Verfahrensablauf zur Verpackung eines Implantats gemäß der vorliegenden Erfindung beschrieben. In einem ersten Verfahrensschritt S1 kann eine noch nicht verschlossene Verpackung, im Wesentlichen umfassend einen erfindungsgemäßen Verpackungskörper, bereitgestellt werden. In einem nachfolgenden Verfahrensschritt S2 kann ein Implantat bereitgestellt werden. In einem Teilverfahrensschritt S2.1 kann dabei vorgesehen sein, dass eine elektrisch leitende und mechanische, insbesondere funktionsbereite, Verbindung zwischen der Kontaktelektrode und dem restlichen Implantat hergestellt wird, zum Beispiel indem ein Elektrodendraht mitsamt der Kontaktelektrode mit einem Gehäuse des Implantats verbunden wird. In einem nachfolgenden Verfahrensschritt S3 kann das Implantat mit der verbundenen Kontaktelektrode in dem noch offenen oder unverschlossenen Verpackungskörper angeordnet werden. Dazu kann beispielsweise das Gehäuse, der Elektrodendraht und die Kontaktelektrode anhand von Führungsmitteln platziert und/oder ausgerichtet werden.

In einem weiteren Verfahrensschritt S4 kann vorgesehen sein, dass durch die Verschlussmittel entlang der Siegelkontur der Verpackungskörper so verschlossen wird, dass ein steriler oder sterilisierbarer Innenraum entsteht, in dem das Implantat mitsamt der Kontaktelektrode angeordnet ist. Das Verschließen oder Versiegeln der Verpackungskörpers kann dabei so ausgestaltet sein, dass ein dauerhafter oder nachträglicher Kontakt zwischen Kontaktelektrode 15 und den Kontaktmitteln 08, wie oben bereits beschrieben, hergestellt wird oder herstellbar ist.

In einem nachfolgenden Verfahrensschritt S5 kann beispielsweise die Sterilisation des Verpackungskörpers mitsamt dem eingeschlossenen Implantat stattfinden.

In einem weiteren Verfahrensschritt S6 kann beispielsweise eine Funktionsüberprüfung des Implantats mitsamt der Kontaktelektrode über eine externe Kontrolleinrichtung über entsprechende Drahtloskommunikationsschnittstellen stattfinden. Die Funktionsprüfung kann bevorzugt beliebig oft wiederholt werden und insbesondere auf Seiten des Herstellers/Verpackers durchgeführt werden und zusätzlich auf Seiten des Operationsteams oder des Operateurs durchgeführt werden, bevor oder unmittelbar bevor die Implantation stattfinden soll und die Verpackung oder der Verpackungskörper geöffnet und der steril verpackte Zustand aufgehoben werden soll.

### Bezugszeichenliste:

- 01: Verpackungskörper
- 02: Verpackungsmaterial
- 03: Verschlussmittel
- 04: Bodenteil
- 05: Deckelteil
- 06: Übergangsbereich
- 07: Führungsmittel
- 08: Kontaktmittel
- 09: Anpressmittel
- 10: Siegelkontur
- 11: Innenraum
- 12: Implantat
- 13: Hauptgehäuse
- 14: Elektrodenkabel
- 15: Kontaktelektrode
- 16: Einzelelektroden
- 17: Verpackungssystem
- 18: Kontrolleinrichtung
- 19: Drahtloskommunikationsschnittstelle
- 20: Führungskonturen
- 21: Trenn- oder Schutzschicht
- 22: Verformungslinie

- S1-S6: Verfahrensschritt

## Patentansprüche

1. Verpackungskörper für ein medizinisches Implantat (12), insbesondere einen Schrittmacher und/oder einen Neurostimulator, mit einer Kontaktelektrode (15), umfassend Verpackungsmaterial (02), welches entlang einer Siegelkontur (10) des Verpackungsmaterials (02) verlaufende Verschlussmittel (03) umfasst, wobei wenigstens ein Teil des Verpackungsmaterials (02) und die Verschlussmittel (03) einen sterilen und/oder sterilisierbaren Innenraum (11) des Verpackungskörpers (01) begrenzen, in dem das Implantat (12) in einem steril verpackten Zustand aufgenommen ist
**dadurch gekennzeichnet,**
**dass** der Verpackungskörper (01), insbesondere im Innenraum (11), elektrisch leitende Kontaktmittel (08) aufweist, welche so angeordnet sind, dass die Kontaktelektrode (15) des Implantats (12) in dem steril verpackten Zustand daran zur Anlage kommt oder zur Anlage bringbar ist.

2. Verpackungskörper nach Anspruch 1,
**dadurch gekennzeichnet,**
**dass** die Kontaktmittel (08) so ausgebildet und/oder angeordnet sind, dass die Kontaktelektrode (15) im steril verpackten Zustand direkt und dauerhaft an dem Kontaktmittel (08) anliegt.

3. Verpackungskörper nach Anspruch 1,
**dadurch gekennzeichnet,**
**dass** die Kontaktmittel (08) beweglich in dem Innenraum (11) angeordnet sind und in eine Kontaktposition binrgbar sind, in welcher die Kontaktelektrode (15) anliegt, ohne dass der steril verpackte Zustand aufgehoben werden muss.

4. Verpackungskörper nach Anspruch 1 oder 3,
**dadurch gekennzeichnet**,
durch eine entfernbare Trenn- oder Schutzschicht (21), die zwischen Kontaktelektrode (15) und Kontaktmitteln (08) angeordnet ist, und die so ausgeführt ist, dass eine Entfernung und eine daraus resultierende Anlage der Kontaktmittel (08) an der Kontaktelektrode (15) möglich ist, ohne dass der steril verpackte Zustand aufgehoben werden muss.

5. Verpackungskörper nach einem der vorangehenden Ansprüche,
**gekennzeichnet durch**
Führungsmittel (07), insbesondere Führungskonturen, im Innenraum (11), die die Lage und Ausrichtung des Implantats (12) und/oder der Kontaktelektrode (15) im verpackten Zustand vorgeben.

6. Verpackungskörper nach einem der vorangehenden Ansprüche,
**gekennzeichnet durch**
Anpressmittel (09), die im steril verpackten Zustand die Kontaktelektrode (15) in Richtung der Kontaktmittel (08) kraftbeaufschlagen.

7. Verpackungskörper nach einem der vorangehenden Ansprüche,
**dadurch gekennzeichnet,**
**dass** die Kontaktmittel (08) metallisches Material umfassen.

8. Verpackungskörper nach einem der vorangehenden Ansprüche,
**dadurch gekennzeichnet,**
**dass** die Kontaktmittel (08) eine Leitfähigkeit aufweisen, die der von lebendem, insbesondere menschlichen, Gewebe entsprechen.

9. Verpackungskörper nach einem der vorangehenden Ansprüche,
**dadurch gekennzeichnet,**
**dass** die Kontaktmittel (08), insbesondere optische, Leitfähigkeitsanzeigemittel umfassen, die eine Abweichung von einem vorgegebenen oder ursprünglichen Leitwert anzeigen.

10. Verpackungskörper nach einem der vorangehenden Ansprüche,
**dadurch gekennzeichnet**,
die Kontaktmittel (08) so ausgebildet sind, dass mehrere, insbesondere alle, Einzelelektroden (16) der Kontaktelektrode (15) im steril verpackte Zustand in Kontakt mit den Kontaktmitteln (08) sind oder bringbar sind.

11. Verpackungssystem zur sterilen Verpackung eines medizinischen Implantats, umfassend das Implantat (12), insbesondere einen Schrittmacher und/oder einen Neurostimulator, mit einer Kontaktelektrode (15),
**gekennzeichnet durch**
einen Verpackungskörper (01) nach einem der Ansprüche 1 bis 10, in dem das Implantat (12) aufgenommen ist.

12. Verpackungssystem nach Anspruch 11,
**dadurch gekennzeichnet,**
**dass** im steril verpackten Zustand die Kontaktelektrode (15) elektrisch leitend, und insbesondere mechanisch, besonders bevorzugt funktionsbereit, mit dem Implantat (12) verbundenen ist.

13. Verpackungssystem nach Anspruch 11 oder 12
**gekennzeichnet durch**,
eine Kontrolleinrichtung (18) mit einer Drahtloskommunikationsschnittstelle (19), die zum Aufbau einer Kommunikationsverbindung mit dem Implantat (12), bevorzugt über eine Drahtloskommunikationsschnittstelle (19) des Implantats (12) eingerichtet ist.

14. Verpackungssystem nach Anspruch 13,
**dadurch gekennzeichnet,**
**dass** die Kontrolleinrichtung (18) zur Funktionsüberprüfung des Implantats (12) einschließlich einer Funktionsüberprüfung der Kontaktelektrode (15) bei bestehender Kommunikationsverbindung mit dem Implantat (12) eingerichtet ist.

15. Verfahren zur sterilen oder sterilisierbaren Verpackung eines Implantats umfassend die Verfahrensschritte:
- Anordnen des Implantats (12), insbesondere eines Schrittmachers und/oder eines Neurostimulators, mit einer Kontaktelektrode (15) in einem Verpackungsmaterial (02);
- Ausbilden einer Siegelkontur (10) im Verpackungsmaterial (02) über Verschlussmittel (03), wodurch wenigstens ein Teil des Verpackungsmaterials (02) und die Verschlussmittel (03) einen sterilen und/oder sterilisierbaren Innenraum (11) eines Verpackungskörpers (01) begrenzen, in dem das Implantat (12) aufgenommen ist
**dadurch gekennzeichnet,**
**dass** die Kontaktelektrode (15) des Implantats (12) so bezüglich des Verpackungsköpers (01), insbesondere im Innenraum (11), angeordnet und/oder ausgerichtet wird, dass in einem steril verpackten Zustand der Verpackungskörper (01) ein elektrisch leitendes Kontaktmittel (08) des Verpackungskörpers (01) an der Kontaktelektrode (15) zur Anlage kommt oder bringbar ist.

16. Verfahren nach Anspruch 15,
**dadurch gekennzeichnet,**
**dass** die Kontaktelektrode (15) mit dem Implantat (12) elektrisch leitend, und insbesondere mechanisch, besonders bevorzugt funktionsbereit, mit dem Implantat (12) verbundenen wird, bevor die Siegelkontur (10) über die Verschlussmittel (03) ausgebildet wird.

17. Verfahren nach Anspruch 15 oder 16,
**dadurch gekennzeichnet,**
**dass** im steril verpackten Zustand eine Funktionsüberprüfung des Implantats (12) einschließlich einer Funktionsüberprüfung der Kontaktelektrode (15) mittels einer drahtlos mit dem Implantat (12) verbundenen Kontrolleinrichtung (18) durchgeführt wird.
